# EUROPÄISCHE PATENTSCHRIFT

(11) **EP 2 489 409 B1**
(45) Veröffentlichungstag und Bekanntmachung des Hinweises auf die Patenterteilung: **10.04.2019**
(21) Anmeldenummer: 12000932.9
(22) Anmeldetag: 14.02.2012
(51) Int. Cl.: A62B 18/08, A61M 16/06, A44B 11/26, A44B 11/25

(54) **Befestigungsvorrichtung für eine Bänderung**
Mounting device for a banding
Dispositif de fixation pour un rubanement

(30) Priorität: 17.02.2011 DE 102011011481
(43) Veröffentlichungstag der Anmeldung: 22.08.2012
(73) Patentinhaber: Löwenstein Medical Technology S.A., 2557 Luxembourg (LU)
(72) Erfinder: Eifler, Martin, 25348 Glückstadt (DE)
(74) Vertreter: Marx, Thomas

(56) Entgegenhaltungen:
- FR-A3- 2 634 358
- US-A- 3 336 641
- US-A- 4 831 694
- US-A- 5 379 496
- US-A1- 2008 230 069

## Beschreibung

Die Erfindung betrifft eine Befestigungsvorrichtung. Die Befestigungsvorrichtung ist insbesondere im Bereich der Medizintechnik einsetzbar, bevorzugt für eine schnell lösbare Befestigung zwischen einer Kopfbänderung bzw. Kopfhaube und einer Beatmungsmaske.

Befestigungsvorrichtungen für die Bänderung einer Beatmungsmaske sind bereits aus dem Stand der Technik bekannt. Insbesondere Befestigungsmittel für Bänderungen bzw. Hauben, die mittels Klettverbindung mit einer Öse verbunden und am Maskenkörper und/oder der Stirnstütze befestigt werden haben eine weite Verbreitung. Die Befestigungsvorrichtung aus der EP1360971B1 weist einen Clip bestehend aus seitlichen Federlaschen, mit Bedienflächen, und einer Öse auf, an welcher die Bänderung befestigt ist. Der Clip wird zum Schließen der Befestigungsvorrichtung mit den Federlaschen in eine Hülse geführt, welche seitliche Aussparungen für die Bedienflächen der Federlaschen aufweist. Zum Lösen der Verbindung muss der Anwender die Bedienflächen der Federlaschen zunächst zusammendrücken, um die Verrastung zu lösen und dann den Clip, unter Beibehaltung des Druckes auf die Federlaschen, aus der Hülse ziehen zu können. Nach demselben Prinzip funktioniert die Lösung aus der US2005199240A1. Es sind damit zwei separate Bewegungen, notwendig, um die Verbindung zwischen Clip und Hülse zu lösen. Dies ist unpraktisch da die Befestigungsvorrichtung für den Anwender nicht im Gesichtsfeld liegt und der Anwender somit die Bewegungsvorgänge zum Lösen nicht unter Sichtkontrolle durchführen kann. Auch andere Lösungen, bei denen die Verbindung über Haken und Ösen realisiert werden, weisen den geschilderten Nachteil auf.

Aus der US 4 831 694 A1 ist zudem eine Schnalle mit einem männlichen Stück und einem weiblichen Stück bekannt, wobei das weibliche Stück ein paar elastische Eingriffsstäbe aufweist und das weibliche Stück in einer flachen zylindrischen Form ausgebildet ist. Aus der US 3 336 641 A ist eine Schnalle mit einem weiblichen und einem männlichen Stück bekannt, wobei das weibliche Stück an einem Gürtelende befestigt ist und das männliche Stück mit einem anderen Bandende verbunden ist. Aus der US 5 379 496 A ist eine Schnurfreigabe-Schnalle mit einem zylindrischen weiblichen Element mit einer zentralen Bohrung und diametral gegenüberliegenden Öffnungen sowie ein zylindrisches männliches Element mit einem Paar Federarme mit einem hakenförmigen Kopf an ihren freien Enden. FR 2 634 358 A3 zeigt eine Nistsicherheitsverschlussvorrichtung mit einem flachen Gehäuse, in dem ein flacher Schieber längs beweglich ist. US 2008/230069 A1 zeigt einen Mechanismus zum Freigeben einer Maske aus dem Gesicht des Patienten, umfassend einen Rahmenteil mit einem männlichen Verbindungsstück und einem weiblichen Verbindungsstück, sowie eine Schnur, die an einem Ende des männlichen Verbindungsstücks angebracht ist und eingerichtet ist, durch ein Ziehen an der Schnur eine Lösung vom weiblichen Verbindungsstück herzustellen.

Die Aufgabe der Erfindung ist es deshalb eine sichere Befestigungsvorrichtung im Bereich eines Maskenkörpers bereitzustellen, die eine deutlich vereinfachte Handhabung beim Lösen der Verbindung bietet.

Insbesondere ist es eine Aufgabe der Erfindung eine Befestigungsvorrichtung im Bereich eines Maskenkörpers bereitzustellen, bei der für das Lösen der Verbindung vom Benutzer lediglich eine Kraftaufwendung in einer definierten Zugrichtung erforderlich ist.

Erfindungsgemäß wird die Aufgabe dadurch gelöst, dass die Befestigungsvorrichtung im Bereich eines Maskenkörpers für eine Bänderung einen Clip mit zumindest einem Federelement aufweist, welches zum verrastenden Eingreifen in einer Aufnahme ausgebildet ist, wobei die Aufnahme im Bereich des Maskenkörpers ausgebildet ist und als Tasche ausgebildet ist, in welcher der Clip verrastet und bei der ein Schieber relativ zum Clip bewegbar ist, wobei eine Bewegung des Schiebers das Federelement aus der Aufnahme bewegt, um die Verrastung zu lösen.

Die Befestigungsvorrichtung besteht aus mindestens drei Teilen, einem Clip und einem Schieber, wobei der Schieber relativ zum Clip verschiebbar ist, sowie einer Aufnahme.

Die Bänderung ist im Bereich einer Bänderungsaufnahme am Clip befestigt. Der Schieber umgreift den Clip zumindest bereichsweise.

Die Aufnahme ist bevorzugt fest oder beweglich mit dem Maskenkörper oder der Stirnstütze verbunden oder als integraler Bestandteil vom Maskenkörper oder der Stirnstütze ausgeführt.

Der Schieber und/oder das Federelement weisen zumindest eine Schräge auf, über welche der Schieber, bei einer Bewegung relativ zum Clip, das Federelement aus seiner Ruhelage auslenkt.

Der Schieber und/oder der Clip weisen mindestens ein Führungselement auf, entlang welchem die Relativbewegung des Schiebers zumindest teilweise erfolgt.

Das mindestens eine Federelement weist mindestens ein Rastelement auf welches im Bereich der Aufnahme an einem Hinterschnitt verrastet.

Der Clip kann zwei seitliche Federelemente aufweisen, deren Rastelemente hinter einen beidseitigen Hinterschnitt in der Aufnahme greifen und so die Verrastung bilden.

Das Federelement gleitet beim Einstecken in die Aufnahme entlang einer Einführschräge und wird durch Keil- und/oder Hebelwirkung ausgelenkt, um so das vollständige Einführen zu ermöglichen.

Der Clip weist einen Führungssteg auf, der zur Führung und Zentrierung im Schieber und/oder in der Aufnahmetasche dient.

Der Clip kann in zwei verschiedenen Orientierungen in die Aufnahm geschoben werde, da die Aufnahme symmetrisch aufgebaut ist und das Federelement in beiden Richtungen aufnehmen kann.

Eine Reißleine an dem Schieber kann als Schnelllösesystem zur Trennung der Verbindung in Paniksituationen dienen.

Das Federelement weist eine schräge Fläche auf, auf die eine Fläche des Schiebers einwirkt, wenn der Schieber in Richtung der Bänderungsöse bewegt wird, zum Lösen der Verbindung.

Zur Montage des Schiebers wird dieser über den Clip geschoben und dabei wird über die schräge Fläche das Federelement aus der Ruhelage ausgelenkt, bis der Schieber seine Position hinter dem Anschlag findet.

Der Clip weist an einem Ende eine Öse als Aufnahme für die Bänderung und in entgegengesetzter Richtung mindestens ein Rastelement auf.

Zum Einführen des Clips in die Aufnahmetasche dienen seitliche Einführschrägen in der Aufnahmetasche die auf die Schrägen der Federelemente einwirken bis die Haken hinter den Flächen der Aufnahme verrasten.

Als Material des Clips, des Schiebers und der Aufnahme wird ein beliebiger, gängiger Hartkunststoff wie beispielsweise POM, PC oder PA gewählt, bevorzugt POM.

Der Schieber enthält als Beschichtung eine Weichkomponente, die komplett oder bereichsweise die Außenfläche des Schiebers umgibt und im 2K-Verfahren mit dem Schieber verbunden oder aufgeklebt ist.

Der Schieber wird nach dem Auslenken des Clips aus der Aufnahme durch die Kräfte aus der elastischen Verformung der/des Schnapphaken in seine Ausgangslage zurückbewegt und/oder dort gehalten.

Bestandteil der Erfindung ist auch ein Clip für die Verwendung in einer erfindungsgemäßen Befestigungsvorrichtung.

Bestandteil der Erfindung ist auch eine Aufnahme für die Verwendung in einer erfindungsgemäßen Befestigungsvorrichtung.

Bestandteil der Erfindung ist auch ein Schieber für die Verwendung in einer erfindungsgemäßen Befestigungsvorrichtung.

Eine leichte Trennung der Verrastung wird dadurch erreicht, dass der Schieber zum Lösen der Verrastung gezogen wird.

Eine einfache Trennung der Verrastung wird auch dadurch erreicht, dass der Schieber zum Lösen der Verrastung geschoben wird.

Eine Aufnahme für die Befestigungsvorrichtung im Bereich des Maskenkörpers bzw. der Stirnstütze ist eine Tasche in welcher der Clip verrastet. Diese Aufnahme kann fest oder beweglich mit dem Maskenkörper oder der Stirnstütze oder einem Koffer, Tasche oder Rucksack verbunden sein oder als integraler Bestandteil von Maskenkörper oder Stirnstützte oder Koffer, Tasche oder Rucksack ausgeführt sein.

Der Clip weist an einem Ende eine Öse als Aufnahme für die Bänderung und in entgegengesetzter Richtung mindestens einen federnden Schnapphaken auf, der in der Aufnahmetasche in einer Öffnung mit Hinterschnitt verrasten kann. Ein Schieber dient als Betätigungselement um die Verrastung des Clips zu lösen.

Dieses wird in den Ausführungsbeispielen und den Figuren beschrieben und dargestellt. Es zeigen:
- Fig. 1: Aufbau einer Befestigungsvorrichtung zum ersten Ausführungsbeispiel
- Fig. 2: Explosionszeichnung der Befestigungsvorrichtung
- Fig. 3: Detaildarstellung des Clips
- Fig. 4: Detaildarstellung des Schiebers
- Fig. 5: Detaildarstellung der Aufnahmetasche
- Fig. 6: Aufbau einer Befestigungsvorrichtung zum zweiten Ausführungsbeispiel
- Fig. 7: Schnittdarstellung der Befestigungsverbindung
- Fig. 8: Detaildarstellung des Clips
- Fig. 9: Detaildarstellung des Schiebers
- Fig. 10: Detaildarstellung des Aufnahmetasche

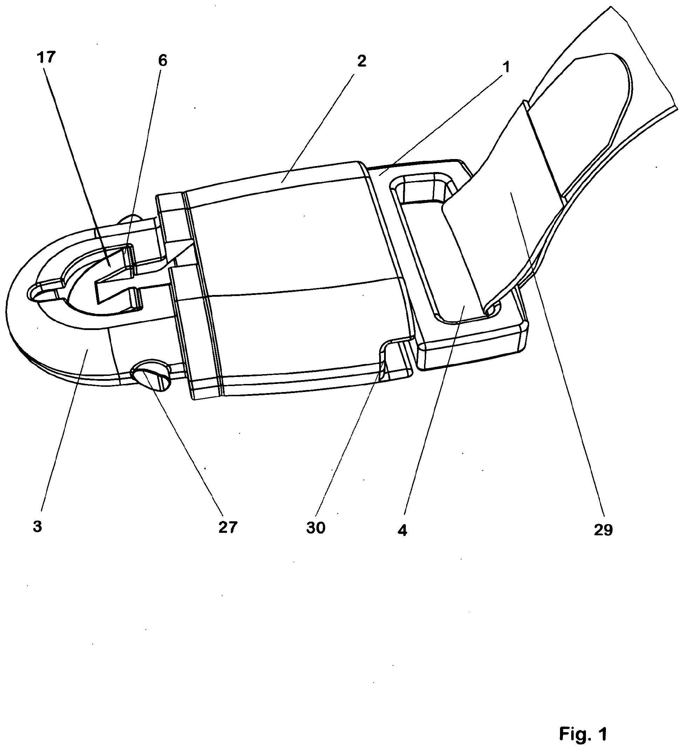

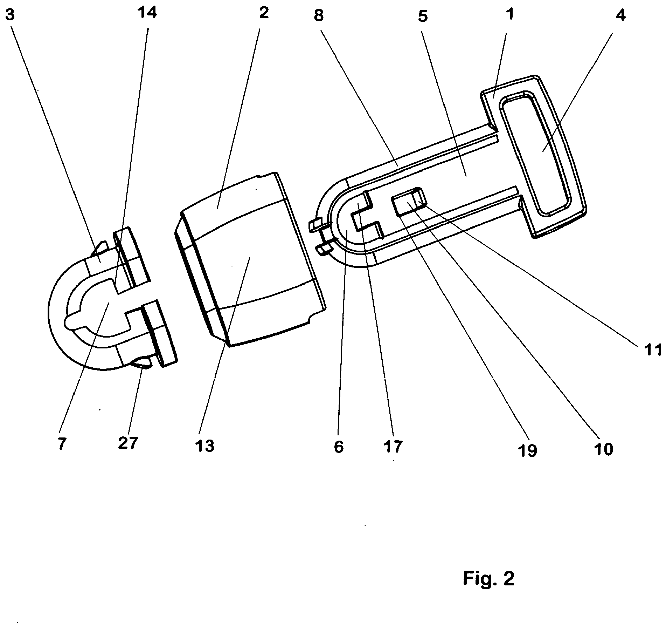

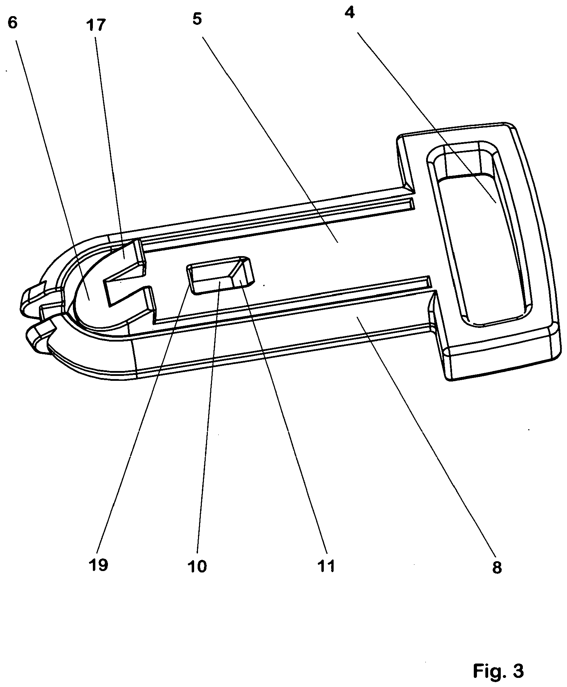

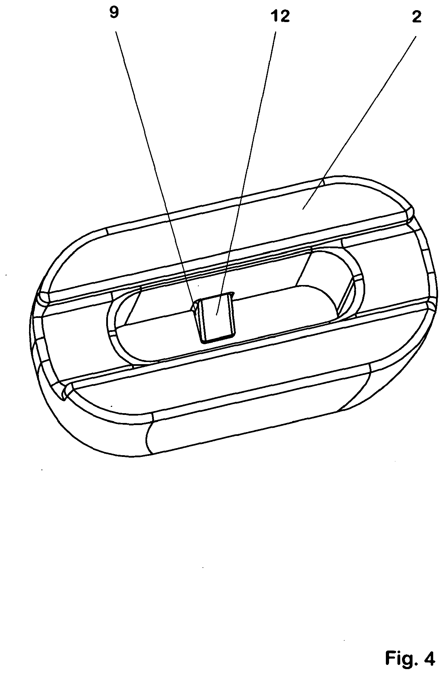

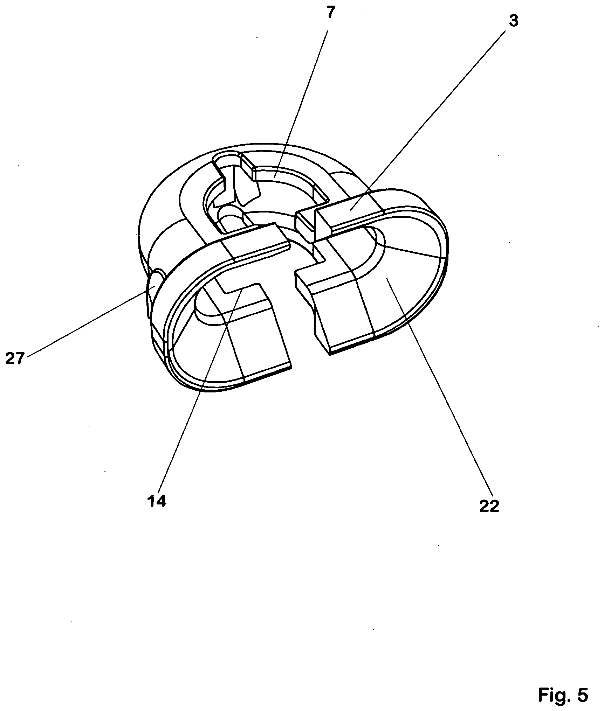

Wie in den Figuren 1 bis 5 dargestellt, weist der Clip (1) als Federelement zumindest einen federnden Schnapphaken (5) auf. Das Rastelement (der Haken) (6), dient der Verrastung in einer Öffnung (7) der Aufnahmetasche (3), Figur 2. Der Schnapphaken (5) wird von einem umlaufenden Steg (8) umschlossen. Der Steg (8) am Clip (1) dient zur Führung und Zentrierung im Schieber (2) und in der Aufnahmetasche (3). Dazu weist der Steg (8) Abmessungen auf, die im Wesentlichen der Öffnung im Schieber (2) entsprechend, welche der Aufnahme des Schnapphakens dient. Der Schnapphaken selbst ist dünner ausgeführt als der Steg (8) und nur im Bereich der Basis des Clips (1) nahe der Bänderungsaufnahme (4) mit dem Clip verbunden. Durch die Wahl des Materials, bevorzugt Kunststoff, werden dadurch federnde Eigenschaften des Schnapphakens (5) bereitgestellt. Der Clip (1) weist eine Bänderungsaufnahme (4) auf, durch die eine, üblicherweise textile, Bänderung (29) geführt werden kann. Die Bänderung kann auch fest mit dem Clip verbunden sein und/oder beispielsweise aus Kunststoff bestehen.

Der Schieber (2) wird über den Clip (1) geschoben und dient als Betätigungselement zum Lösen der Schnappverbindung.

Der Schieber (2) weist dazu innen eine Nase (9) auf, die in eine Öffnung (10) im Schnapphaken (5) greift. Die Öffnung (10) im Schnapphaken (1) weist an einer Seite eine Schräge (11) auf. Die Nase (9) im Schieber (2) ist ebenfalls mit einer Schräge (12) versehen. Diese Schrägen (11, 12) dienen als schiefe Ebene zum Einkoppeln und Auskoppeln der Schnappverbindung, wenn der Schieber (2) in Richtung Bänderungsöse (4) gezogen/geschoben wird. Hierbei gleiten die Schrägen (11,12) übereinander und üben so eine Kraft auf den Schnapphaken (5) aus, der aus seiner Ruhelage ausgelenkt wird. Das gleiche Ergebnis kann aber auch erzielt werden, wenn man Nase (9) und Öffnung (10) vertauscht. Im Schieber (2) ist eine Nase (9) und im Schnapphaken (5) eine Öffnung (10).

Das Verrasten des Clips (1) in der Tasche (3) erfolgt durch einfaches Einstecken. Zum Einstecken kann der Anwender den Schieber (2) oder den Clip (1) als Grifffläche nutzen. Dabei kommt es im Wesentlichen nicht zu einer Relativbewegung zwischen Clip (1) und Schieber (2), da die Bewegung des Schiebers (2) auf dem Clip in Richtung Aufnahmetasche (3) durch die Nase (9), welche in der Öffnung (10) verrastet, begrenzt wird.

Beim Einstecken des Clips (1) in die Tasche (3) gleitet das als Haken (6) ausgeführte Rastelement (6) mit seiner Einführschräge entlang einer korrespondierenden Schräge an der Öffnung der Tasche (3) nach unten. Der Schnapphaken (5) wird somit durch Keil- und Hebelwirkung kurzzeitig ausgelenkt und ermöglicht das vollständige Einführen, bis der Haken (6) im Bereich der Öffnung (7) an einem Hinterschnitt einrastet.

Ist der Clip (1) in der Aufnahmetasche (3) verrastet, kann diese Verbindung nicht durch Zugkräfte auf den Clip (4) gelöst werden, sondern bietet einen sicheren Halt der Verbindung.

Zum Lösen der Verbindung muss der Schieber (2) in Richtung Bänderungsaufnahme (4) geschoben/gezogen werden. Durch ein Verschieben des Schiebers (2) relativ zum Clip (1) wird der Schnapphaken (5) ausgelenkt, so dass es zum Lösen der Verbindung zwischen Haken (6) und Hinterschnitt der Öffnung (7) kommt. Das Auslenken wird durch Hebel- oder Keilwirkung, die die Schrägen (11,12) auf den Schnapphaken ausüben, verursacht. Der Schnapphaken (5) wird dabei indirekt betätigt. Seitliche Ausnehmungen (30) am Schieber (2) bilden zusammen mit der Bänderungsaufnahme (4) einen Endanschlag bei der Bewegung des Schiebers, sodass der Schieber nicht über die Bänderungsaufnahme (4) geschoben werden kann.

Die Betätigungsrichtung des Schiebers (2) entspricht im Wesentlichen der Richtung, mit der der Clip in die Tasche geschoben wird. Die Zugrichtung der Bänderung kann von dieser Richtung abweichen. Um eine optimale Anpassung der Maske zu ermöglichen, kann die Aufnahmetasche (3) am Maskenkörper (26) bzw. der Stirnstütze (26') beweglich gelagert sein. Der Maskenkörper (26) weist dazu eine Aufnahme (28) für die Tasche (3) auf. Die Tasche verrastet dort mit ihren Rastelementen (27). Bedingt durch den Aufbau der Tasche (3) in U-Form und bedingt durch das flexible Material, kann die Tasche durch leichten Druck im Bereich der Rastelementen (27) verformt werden und so leicht in die Aufnahme (28) im Maskenkörper (26) eingeführt werden.

Der Schieber (2) kann Griffmulden oder Griffflächen (13) aufweisen, um dem Anwender einen sicheren Halt beim Betätigen des Schiebers (2) zu gewährleisten.

Der Clip (1) kann in zwei verschiedenen Orientierungen in die Tasche (3) geschoben werden, da die Tasche (3) symmetrisch aufgebaut ist und den Schnapphaken (5) des Clips (1) in beiden Richtungen aufnehmen kann. Es ist nur darauf zu achten, dass die Bänderung, die in die Bänderungsaufnahme (4) des Clips (1) eingefädelt ist, nicht verdreht ist, da ansonsten Druckstellen am Patientengesicht entstehen können.

Optional kann eine Reißleine an einem der Schieber (2) befestigt sein. Diese dient zum schnellen Trennen der Verbindung z.B. in Paniksituationen. Da man den rechten und linken Clip (1) vertauschen kann, besteht die Möglichkeit das Schnelllösesystem mit nur einer Reißleine sowohl für Rechtshänder als auch für Linkshänder zu ermöglichen.

In der Anwendung ist der Schieber (2) über den Führungssteg (8) des Clips (1) geschoben und dort über die Nase (9) im Schieber (2), die in die Öffnung (10) im Schnapphaken (5) greift, gerastet. Diese Verbindung kann aber zum Reinigen der Einzelteile zerlegt werden. Es genügt dazu ein leichter Druck auf den Schnapphaken (5) und die Verrastung der Nase (9) des Schiebers (2) in der Öffnung (10) des Schnapphakens (5) löst sich. Zur Montage wird der Schieber (2) wieder über den Clip (1) geschoben.

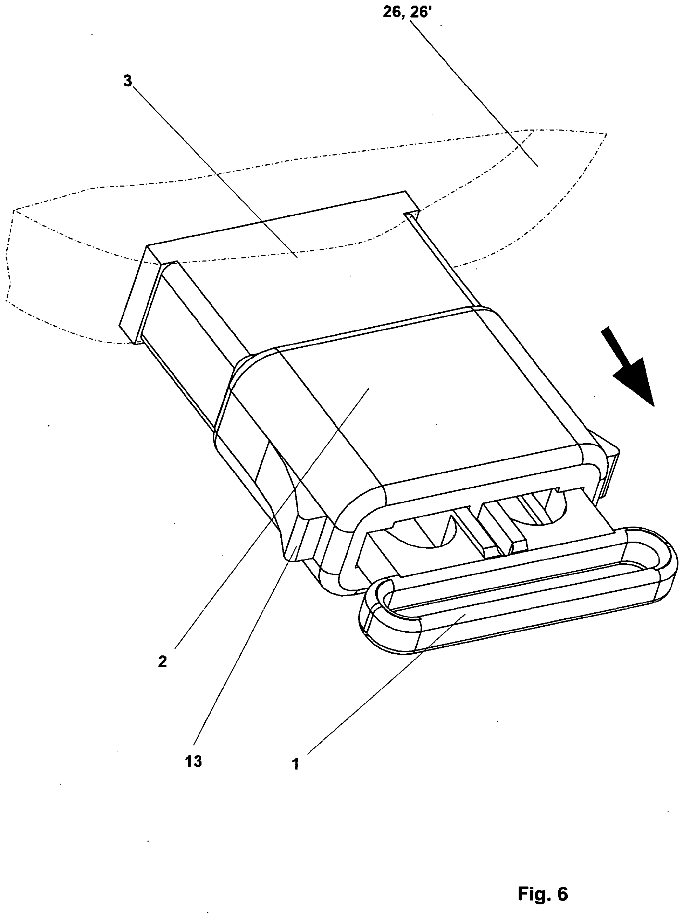

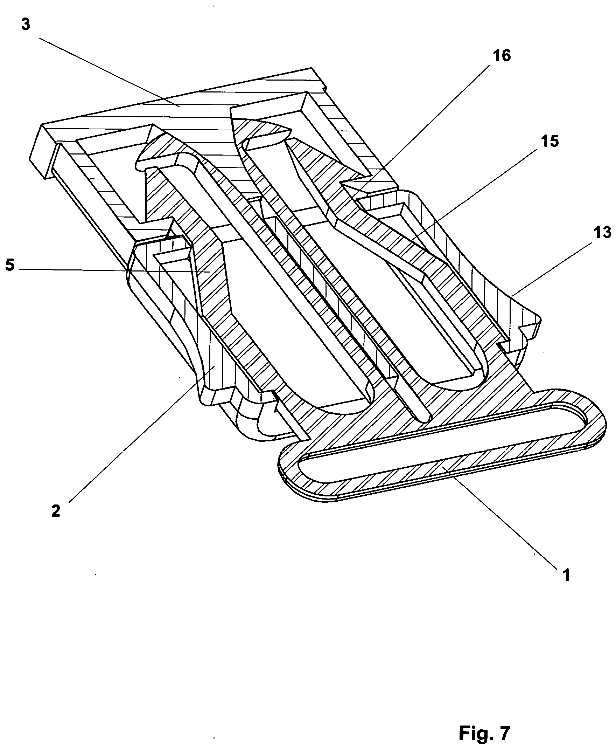

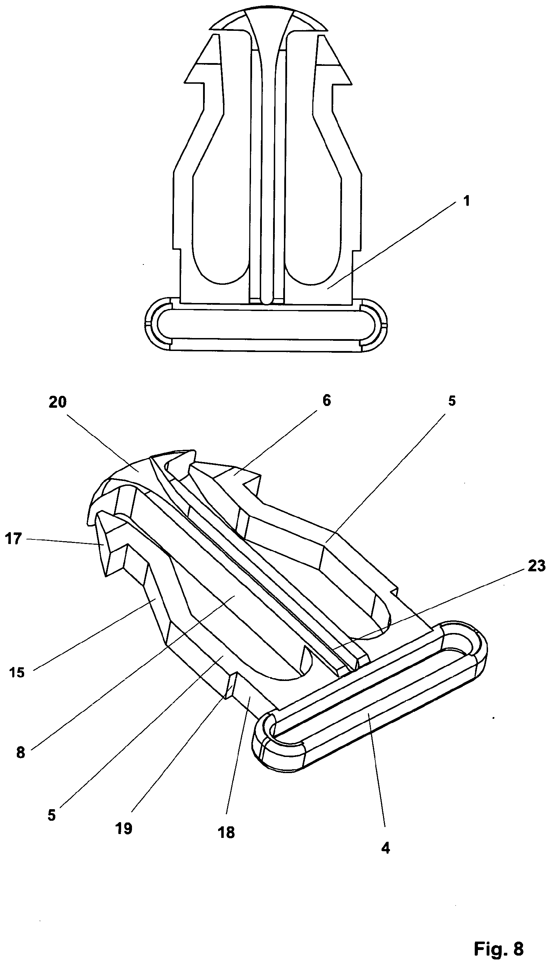

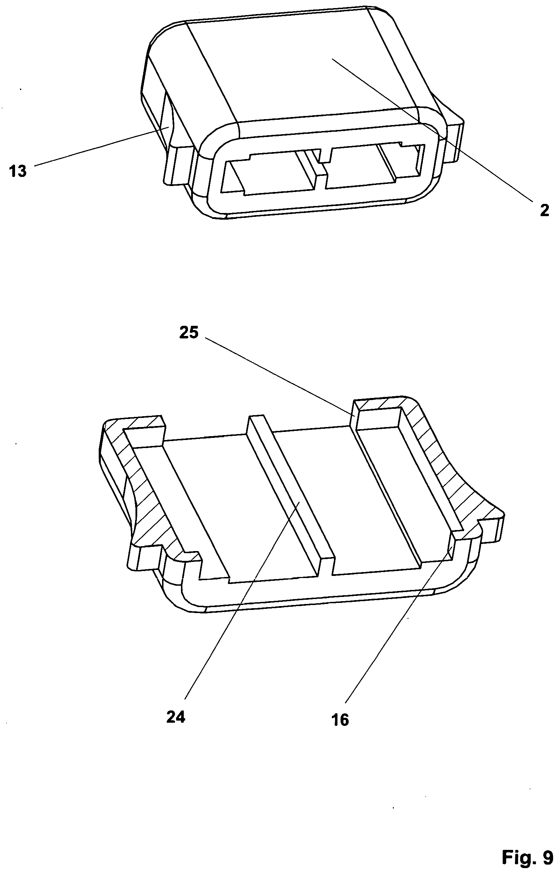

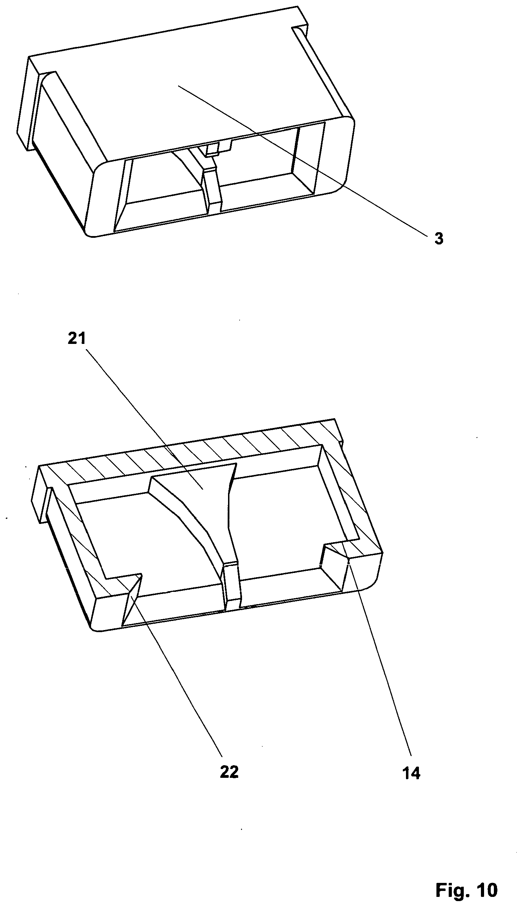

Im den Fig. 6 bis Fig. 10 weist der Clip (1) zwei federnde Schnapphaken (5) auf. Diese beiden Schnapphaken (5) liegen seitlich außen am Clip (1). Zur Führung und Zentrierung dient ein Steg (8) in der Mitte des Clips (1), welcher dicker ausgebildet ist als die Schnapphaken.

Die federnden Schnapphaken (5) weisen am Ende Rastelemente (6) auf, die bei der Montage hinter einen beidseitigen Hinterschnitt (14) in der Aufnahmetasche (3) greifen und so die Verbindung halten. Die Schnapphaken (5) weisen zudem jeweils eine schräge Fläche (15) auf. Auf diese Schräge (15) wirkt die Nase (9, 25) des Schiebers (2) ein, wenn dieser in Richtung der Bänderungsöse (4) gezogen/geschoben wird und löst dabei die Verbindung der Haken (6) der Schnapphaken (5) am Hinterschnitt (14) der Tasche (3).

Zur Montage des Schiebers (2) wird dieser über den Clip (1) geschoben. Dabei wird der Schieber (2) über die schrägen Flächen (17) an den Haken (6) der Schnapphaken (5) geführt und drückt dabei die Schnapphaken (5) leicht zusammen. In dieser Position bilden zum Einen die Bänderungsöse (4) und zum Anderen seitliche Verjüngungen (18) an den Schnapphaken (5) einen Anschlag (19) für den Schieber (2), sodass dieser nicht vom Clip (1) rutschen kann.

Der Führungssteg (8) in der Mitte des Clips (1) weist am Ende eine Öffnung (20) auf, in die ein Gegenstück (21) der Aufnahmetasche (3) zur Führung und Zentrierung greift. An der Ober- und Unterseite angebrachte Nuten (23) im Führungssteg (8) dienen der Führung des Schiebers (2), der innen an Ober- und Unterseite Stege (24) aufweist, die in den Nuten (23) geführt werden. Der Schieber (2) besitzt außen Griffmulden (13) zur einfachen und sicheren Betätigung des Schiebers (2). Beim Betätigen des Schiebers (2) zum Lösen der Verbindung in der Aufnahmetasche (3) drückt der Schieber mit der Nase (9, 25) auf die seitliche schräge Fläche (15) am Schnapphaken (5) und lenkt so die Schnapphaken (5) - durch Keil-und Hebelwirkung - aus und löst die Verbindung. Danach wird der Schieber (2) - durch die Kräfte aus der elastischen Verformung der Schnapphaken - in seine Ausgangslage zurückbewegt und dort gehalten.

Als Einführhilfe zur Montage des Clips (1) in die Aufnahmetasche (3) dienen seitliche Einführschrägen (22) in der Aufnahmetasche (3).

Der Clip (1) und der Schieber (2) sind symmetrisch ausgeführt, sodass die Verbindung in zwei verschiedenen Orientierungen in die ebenfalls symmetrische Aufnahmetasche (3) gesteckt werden kann. Auch hier kann ebenfalls optional eine Reißleine zur Trennung der Verbindung für Rechts- bzw. Linkshänder angebracht sein.

Beide aufgeführten Ausführungsbespiele funktionieren nach dem gleichen Prinzip und bieten dem Anwender eine leichte und sichere Anwendung beim Öffnen und Schließen der Bänderung am Maskenkörper und/oder der Stirnstütze und einen sicheren Halt der Maske am Kopf des Anwenders.

Als Material des Clips (1), des Schiebers (2) und der Aufnahmetasche (3) kann ein beliebiger, gängiger Hartkunststoff gewählt werden, wie z. B. POM, PC oder PA gewählt, bevorzugt POM. Der Schieber (3) kann zusätzlich als Beschichtung oder haptisches Element eine Weichkomponente enthalten, die komplett oder bereichsweise die Außenfläche des Schiebers (3) umgibt. Diese Weichkomponente kann im 2K-Verfahren mit dem Schieber verbunden oder aufgeklebt sein. Diese Art der Befestigungsvorrichtung kann natürlich auch andere Einsatzgebiete z.B. im Bereich der Medizintechnik oder dem Consumer-Bereich finden. Es können z.B. Schnallen oder Verschlüsse zum Verschließen von Sanitätskoffern, -rucksäcken aber auch für alle anderen Arten von Taschen, Koffern oder Rucksäcken (26") nach dem gleichen Prinzip funktionieren.

### Positionsnummern:

- 1: Clip
- 2: Schieber
- 3: Aufnahme
- 4: Bänderungsaufnahme
- 5: Schnapphaken, Federelement
- 6: Haken, Rastelement
- 7: Öffnung in der Aufnahmetasche (3)
- 8: Führungssteg
- 9: Fläche zum Auslenken der Schnapphaken, Nase
- 10: Öffnung im Schnapphaken, Federelement (5)
- 11: Schräge in der Öffnung (10)
- 12: Schräge an der Nase (9)
- 13: Griffmulde oder Grifffläche am Schieber (2)
- 14: Hinterschnitt in der Aufnahmetasche (3)
- 15: Schräge Flächen am Schnapphaken (5)
- 16: Raststelle für Anschlag (19)
- 17: Schräge an den Haken (6)
- 18: Verjüngung am Schnapphaken
- 19: Anschlag für Schieber (2)
- 20: Öffnung im Führungssteg (8)
- 21: Führung in der Aufnahmetasche (3)
- 22: Einführschrägen in der Aufnahmetasche (3)
- 23: Nuten im Führungssteg (8)
- 24: Führungsstege im Schieber (2)
- 25: Fläche zum Auslenken der Schnapphaken, Nase
- 26, 26', 26": Maskenkörper, Stirnstütze, Rucksack, Koffer
- 27: Rastelement an Aufnahmetasche (3)
- 28: Aufnahme der Aufnahmetasche (3) im Maskenkörper, Stirnstütze, Rucksack, Koffer, Tasche
- 29: Bänderung
- 30: Endanschlag

## Patentansprüche

1. Befestigungsvorrichtung im Bereich eines Maskenkörpers für eine Bänderung (29) aufweisend einen Clip (1) mit zumindest einem Federelement (5), welches zum verrastenden Eingreifen in einer Aufnahme (3) ausgebildet ist, **dadurch gekennzeichnet, dass** die Aufnahme (3) im Bereich des Maskenkörpers ausgebildet ist und als Tasche ausgebildet ist, in welcher der Clip (1) verrastet und ein Schieber (2) relativ zum Clip (1) bewegbar ist, wobei eine Bewegung des Schiebers (2) das Federelement (5) aus der Ruhelage bewegt, um die Verrastung zu lösen.

2. Befestigungsvorrichtung nach Anspruch 1, **dadurch gekennzeichnet, dass** der Schieber (2) den Clip (1) zumindest bereichsweise umgreift.

3. Befestigungsvorrichtung nach einem der vorhergehenden Ansprüche **dadurch gekennzeichnet, dass** der Schieber (2) und/oder das Federelement (5) zumindest eine Schräge (11, 12, 15) aufweisen, über welche die Nase (9, 25) bei einer Bewegung relativ zum Clip (1), das Federelement (5) aus seiner Ruhelage auslenkt.

4. Befestigungsvorrichtung nach einem der vorhergehenden Ansprüche, **dadurch gekennzeichnet, dass** der Schieber (2) und/oder der Clip (1) mindestens ein Führungselement (8, 23, 24) aufweisen, entlang welchem die Relativbewegung des Schiebers (2) zumindest teilweise erfolgt.

5. Befestigungsvorrichtung nach einem der vorhergehenden Ansprüche, **dadurch gekennzeichnet, dass** das mindestens eine Federelement (5) mindestens ein Rastelement (6) aufweist welches im Bereich der Aufnahme (3) an einem Hinterschnitt (14) verrastet.

6. Befestigungsvorrichtung nach einem der vorhergehenden Ansprüche, **dadurch gekennzeichnet, dass** der Clip (1) das zumindest eine Federelement (5) aufweist, dessen Rastelement (6) hinter einem Hinterschnitt (14) in der Aufnahme (3) greift und so die Verrastung bildet.

7. Befestigungsvorrichtung nach einem der vorhergehenden Ansprüche, **dadurch gekennzeichnet, dass** das Federelement (5) beim Einstecken in die Aufnahme (3) entlang einer Einführschräge (22) gleitet und durch Keil- und/oder Hebelwirkung ausgelenkt wird, um so das vollständige Einführen zu ermöglichen.

8. Befestigungsvorrichtung nach einem der vorhergehenden Ansprüche, **dadurch gekennzeichnet, dass** der Clip (1) in zwei verschiedenen Orientierungen in die Aufnahme (3) geschoben werden kann, da die Aufnahme (3) symmetrisch aufgebaut ist und das Federelement (5) des Clips (1) in beiden Richtungen aufnehmen kann.

9. Befestigungsvorrichtung nach einem der vorhergehenden Ansprüche, **dadurch gekennzeichnet, dass** eine Reißleine an dem Schieber (2) als Schnelllösesystem zur Trennung der Verbindung in Paniksituationen dient.

10. Befestigungsvorrichtung nach Anspruch 4, **dadurch gekennzeichnet, dass** das Federelement (5) eine schräge Fläche (15) aufweist, auf die Nase (9, 25) des Schiebers (2) einwirkt, wenn der Schieber (2) relativ zum Federelement bewegt wird.

11. Befestigungsvorrichtung nach einem der vorhergehenden Ansprüche, **dadurch gekennzeichnet, dass** der Clip (1) an einem Ende eine Öse (4) als Aufnahme für die Bänderung und in entgegengesetzter Richtung mindestens ein Rastelement (6) aufweist.

12. Befestigungsvorrichtung nach einem der vorhergehenden Ansprüche, **dadurch gekennzeichnet, dass** zum Einführen des Clips (1) in die Aufnahmetasche (3) seitliche Einführschrägen (22) in der Aufnahmetasche (3) auf die Schrägen (17) der Federelemente (5) einwirken bis die Haken (6) hinter den Flächen (14) der Aufnahme (3) verrasten.

13. Befestigungsvorrichtung nach einem der vorhergehenden Ansprüche, **dadurch gekennzeichnet, dass** der Schieber (2) als Beschichtung eine Weichkomponente enthält, die komplett oder bereichsweise die Außenfläche des Schiebers (2) umgibt und im 2K-Verfahren mit dem Schieber verbunden oder aufgeklebt ist.

14. Befestigungsvorrichtung nach einem der vorhergehenden Ansprüche, **dadurch gekennzeichnet, dass** der Schieber (2) nach dem Auslenken des Clips (1) aus der Aufnahme (3) durch die Kräfte aus der elastischen Verformung der/des Schnapphaken (5) in seine Ausgangslage zurückbewegt wird und/oder dort gehalten wird.

## Claims

1. A fastening device in the region of a mask body for a harness (29) having a clip (1) with at least one spring element (5) that is designed to latchingly engage in a seat (3), **characterized in that** the seat (3) is formed in the region of mask body and is designed as a pocket in which the clip (1) latches, and a slider (2) can be moved relative to the clip (1), wherein a movement of the slider (2) moves the spring element (5) out of the home position in order to release the latching.

2. The fastening device according to claim 1, **characterized in that** the slider (2) surrounds the clip (1), at least in a region.

3. The fastening device according to one of the preceding claims, **characterized in that** the slider (2) and/or the spring element (5) has at least one chamfer (11, 12, 15) by which the nose (9, 25) disengages the spring element (5) from its home position in a movement relative to the clip (1).

4. The fastening device according to one of the preceding claims, **characterized in that** the slider (2) and/or the clip (1) has at least one guide element (8, 23, 24) along which the relative movement of the slider (2) at least partially occurs.

5. The fastening device according to one of the preceding claims, **characterized in that** the at least one spring element (5) has at least one latching element (6) that latches in an undercut (14) in the region of the seat (3).

6. The fastening device according to one of the preceding claims, **characterized in that** the clip (1) has the at least one spring element (5) with a latching element (6) that engages behind an undercut (14) in the seat (3) and thereby establishes the latching.

7. The fastening device according to one of the preceding claims, **characterized in that** the spring element (5) slides along a lead-in chamfer (22) while being inserted in the seat (3) and is deflected by a wedge and/or lever effect in order to thus enable complete insertion.

8. The fastening device according to one of the preceding claims, **characterized in that** the clip (1) can be shoved into the seat (3) in two different orientations since the seat (3) is designed symmetrical and can accommodate the spring element (5) of the clip (1) in both directions.

9. The fastening device according to one of the preceding claims, **characterized in that** a release cord on the slider (2) serves as a quick release system to disconnect the connection in panic situations.

10. The fastening device according to claim 4, **characterized in that** the spring element (5) has an oblique surface (15) that acts on the nose (9, 25) of the slider (2) when the slider (2) is moved relative to the spring element.

11. The fastening device according to one of the preceding claims, **characterized in that** the clip (1) has an eye (4) at one end as a seat for the harness and at least one latching element (6) in the opposite direction.

12. The fastening device according to one of the preceding claims, **characterized in that**, to introduce the clips (1) into the seat pocket (3), lateral lead-in chamfers (22) in the seat pocket (3) act on the chamfers (17) of the spring elements (5) until the hooks (6) latch behind the surfaces (14) of the seat (3) .

13. The fastening device according to one of the preceding claims, **characterized in that** the slider (2) contains a soft component as a coating that completely or sectionally surrounds the outer surface of the slider (2) and is connected or glued to the slider in a two component method.

14. The fastening device according to one of the preceding claims, **characterized in that**, after the clip (1) is disengaged from the seat (3), the slider (2) is moved back into its home position and/or is held there by the forces arising from the elastic deformation of the snap hook(s) (5).

## Revendications

1. Dispositif de fixation dans la région d'un corps de masque pour un harnais (29) présentant un clip (1) avec au moins un élément élastique (5) conçu pour s'engager par encliquetage dans un logement (3), **caractérisé en ce que** le logement (3) est formé dans la région du corps de masque et formé comme une poche, dans laquelle le clip (1) s'encliquète et un coulisseau (2) peut se déplacer par rapport au clip (1), sachant qu'un déplacement du coulisseau (2) permet de sortir l'élément élastique (5) de la position de repos pour défaire l'encliquetage.

2. Dispositif de fixation selon la revendication 1, **caractérisé en ce que** le coulisseau (2) entoure au moins partiellement le clip (1) .

3. Dispositif de fixation selon l'une des revendications précédentes, **caractérisé en ce que** le coulisseau (2) et/ou l'élément élastique (5) présentent au moins un chanfrein (11, 12, 15), par le biais duquel le nez (9, 25) fait sortir l'élément élastique (5) de sa position de repos lors d'un déplacement par rapport au clip (1).

4. Dispositif de fixation selon l'une des revendications précédentes, **caractérisé en ce que** le coulisseau (2) et/ou le clip (1) présentent au moins un élément de guidage (8, 23, 24), le long duquel le déplacement relatif du coulisseau (2) s'effectue au moins partiellement.

5. Dispositif de fixation selon l'une des revendications précédentes, **caractérisé en ce que** l'au moins un élément élastique (5) présente au moins un élément d'encliquetage (6), lequel s'encliquète sur une contre-dépouille (14) dans la région du logement (3).

6. Dispositif de fixation selon l'une des revendications précédentes, **caractérisé en ce que** le clip (1) présente l'au moins un élément élastique (5), dont l'élément d'encliquetage (6) s'engage dans le logement (3) derrière une contre-dépouille (14), formant ainsi l'encliquetage.

7. Dispositif de fixation selon l'une des revendications précédentes, **caractérisé en ce que** l'élément élastique (5) glisse le long d'un chanfrein d'insertion (22) lors de l'insertion dans le logement (3), tout en étant dévié par un effet de cale et/ou de levier, pour ainsi permettre l'insertion complète.

8. Dispositif de fixation selon l'une des revendications précédentes, **caractérisé en ce que** le clip (1) peut être poussé dans le logement (3) dans deux orientations différentes, puisque le logement (3) est conçu de façon symétrique et peut recevoir l'élément élastique (5) du clip (1) dans les deux sens.

9. Dispositif de fixation selon l'une des revendications précédentes, **caractérisé en ce qu'**un cordon de décrochage sur le coulisseau (2) sert de système de détachement rapide pour défaire la liaison dans des situations de panique.

10. Dispositif de fixation selon la revendication 4, **caractérisé en ce que** l'élément élastique (5) présente une surface oblique (15), laquelle agit sur le nez (9, 25) du coulisseau (2) lorsque le coulisseau (2) est déplacé par rapport à l'élément élastique.

11. Dispositif de fixation selon l'une des revendications précédentes, **caractérisé en ce que** le clip (1) présente un oeillet (4) à une extrémité, en tant que logement pour le harnais, et au moins un élément d'encliquetage (6) dans la direction opposée.

12. Dispositif de fixation selon l'une des revendications précédentes, **caractérisé en ce que** pour l'insertion du clip (1) dans la poche de logement (3), des chanfreins d'insertion latéraux (22) dans la poche de logement (3) agissent sur les chanfreins (17) des éléments élastiques (5) jusqu'à ce que les crochets (6) s'encliquètent derrière les surfaces (14) du logement (3).

13. Dispositif de fixation selon l'une des revendications précédentes, **caractérisé en ce que** le coulisseau (2) contient un composant souple en tant que revêtement, lequel entoure complètement ou partiellement la surface extérieure du coulisseau (2), tout en étant relié ou collé au coulisseau à l'aide d'un procédé 2K.

14. Dispositif de fixation selon l'une des revendications précédentes, **caractérisé en ce que** le coulisseau (2), après la déviation du clip (1) hors du logement (3), est renvoyé vers sa position initiale et/ou maintenu dans celle-ci par les forces issues de la déformation élastique du/des crochet(s) d'enclipsage (5).
